Europäisches Patentamt

European Patent Office

Office européen des brevets

(19)

(11) EP 1 582 590 A1

(12) **EUROPEAN PATENT APPLICATION**

(43) Date of publication:
05.10.2005 Bulletin 2005/40

(51) Int Cl.⁷: **C12N 15/10**, G01N 33/50, C12Q 1/68

(21) Application number: 04290877.2

(22) Date of filing: 02.04.2004

(84) Designated Contracting States:
AT BE BG CH CY CZ DE DK EE ES FI FR GB GR HU IE IT LI LU MC NL PL PT RO SE SI SK TR
Designated Extension States:
AL HR LT LV MK

(71) Applicant: Aptanomics
69364 Lyon Cedex 07 (FR)

(72) Inventors:
• Bickle, Marc
69005 Lyon (FR)
• Borie, Christophe
69007 Lyon (FR)

• Colas, Pierre
69370 Saint Didier au Mont d'Or (FR)

(74) Representative: Almond-Martin, Carol et al
Ernest Gutmann - Yves Plasseraud S.A.
3, rue Chauveau-Lagarde
75008 Paris (FR)

Remarks:
The sequence listing, which is published as annex to the application documents, was filed after the date of filing. The applicant has declared that it does not include matter which goes beyond the content of the application as filed.

(54) **Sequential intracellular screening method**

(57) The present invention relates to an intracellular screening method for detecting exogenous candidate compounds C capable of modulating the intracellular interaction between two peptidic binding partners A and B, or of creating such an interaction, said intracellular interaction being responsible for a given specific phenotype, wherein in a first step, the candidate compound C is allowed to contact the binding partner A, inside a cell, in the absence of the partner B, in a second step, the partner B is then made available for binding to partner A, and in a third step, the phenotype of said cell is de-

tected. The invention also relates to an Intracellular screening method for detecting exogenous candidate compounds C capable of modulating the intracellular interaction between two peptidic binding partners A and B and being incapable of modulating the interaction between peptidic binding partners A' and B', said intracellular interactions being responsible for given specific cellular phenotypes, wherein said method is also based on the sequential exposure of the candidate compound to each one of the binding partners.

EP 1 582 590 A1

**Description**

The present invention relates to a screening method allowing the detection of compounds capable of modulating the intracellular interaction between two peptidic binding partners or capable of creating such an intracellular interaction. The screening method of the invention is based on the sequential exposure of the candidate compound to each one of the binding partners.

Protein interactions represent a huge, largely untapped, reservoir of therapeutic targets. The development of sensitive and robust screening assays is a key factor of success in the discovery of small molecule drugs able to disrupt protein interactions. Moreover, since some diseases involve the breakdown or loss of interactions which normally take place in healthy individuals, it is also important to find compounds capable of generating or restoring specific interactions.

A number of screening methods have been developed aimed at finding such compounds.

For example, a widely used method for detecting modulations of interactions is the so-called "two-hybrid assay" [Golemis, 1997], initially developed in yeast cells; and now having counterparts in bacterial and mammalian cells.

Briefly, in this system, a first nucleic acid sequence is constructed coding for a first peptide A fused to a DNA binding domain of a transcription factor and a second sequence coding for a second peptide B fused to a transcriptional activation domain, wherein the expression of both sequences reconstitutes an active transcription factor when an interaction occurs between peptides A and B. A cell is transformed with both sequences and with the sequence of a reporter gene placed under positive transcriptional control of the reconstituted transcriptional activation domain. When an interaction occurs between peptides A and B, this results in the reconstitution of the active transcription factor, leading to the expression of the reporter gene. Inhibitors of the interaction can be identified on the basis of a decrease in reporter gene expression. This type of assay, where the two hybrid phenotype is expression of a reporter gene which is not toxic to the cell, is generally known as a "forward" two-hybrid assay.

Forward two-hybrid assays are simple, versatile and robust, and can accommodate developments and adaptations relatively easily. For example, a forward two-hybrid assay was recently used successfully to identify an inhibitor of the Ras-Raf1 interaction from a library of more than 73,000 compounds [Kato-Stankiewicz, 2002]. In this study, yeast were plated as a monolayer in soft agar and both growth and β-galactosidase activity were used as two-hybrid phenotypes. This screening proved successful. Compounds that inhibited yeast growth non-specifically were eliminated by carrying out a second parallel screening.

Variants of the two-hybrid assay have also been designed. For example, non-transcriptional two-hybrid assays, referred to as "Repressed transcription assays (RTA)", have been engineered, wherein the interaction between two peptides allows the reconstitution of a repressor, said repressor preventing the constitutive expression of a reporter gene.

The "reverse" two-hybrid assay system is also an example of such a variant [see Vidal, 1999a for a review]. In these reverse assays, the two-hybrid phenotype is absence of growth or death so that a disruption of the protein interaction allows yeast to grow. A reverse two-hybrid assay detected successfully the inhibition of interaction between TGF-β-receptor and FKBP12 by FK506 [Huang, 1997]. 30nM of FK506 was seen to produce a detectable growth on solid medium. Yeast growth caused by the inhibition of protein interaction by FK506 was detected in nanodroplets of liquid medium [Huang, 1997]. Another "reverse" two-hybrid assay was used successfully in a screening of a library of 156,000 molecules, to identify 10 hits inhibiting the interaction between two subunits of a N-type calcium channel [Young, 1998].

These traditional two-hybrid assays allow to screen for compounds capable of inhibiting the interaction between two peptidic partners A and B, interacting in a cell. Indeed, a decrease in the intensity of the phenotypic trait caused by the reporter gene is indicative of an inhibition in the interaction.

However, the known systems often suffer from lack of sensitivity and are not adapted to screen for compounds capable of preventing an interaction from occurring. Moreover, most of them are only capable of detecting compounds which completely disrupt the interaction.

For example, in the classical systems, compounds which weakly modulate the interaction are generally not detected due to lack of sensitivity of the screen. Moreover, when the candidate compound is applied externally to the cell, it generally takes time to arrive at the cellular site where interaction occurs, and is generally in low concentrations. Therefore, the candidate compound is at a disadvantage in comparison with the interacting partners.

It is also an inconvenience of the known methods that candidate compounds which are capable of preventing the interaction between peptides A and B, for example by occupying the binding surface of one of them, are not detected if these compounds cannot disrupt the interaction once it has occurred, for example because the dissociation rate constant $k_{diss}$ of the complex A-B is unfavourable, because the dissociation of the complex A-B is very slow (i.e. the $k_{diss}$ rate constant is smaller than $10^{-5}$ s$^{-1}$).

There is thus a need for a screening method which allows the detection of compounds which are capable of modulating a known intracellular peptidic interaction or capable of creating a peptidic interaction. There is moreover a need for a screening method which allows detection of weak modifications of the interaction, a method which is rapid, sensitive, with a wide dynamic range, specifically, the method must be capable of detecting a small modulation in a very strong interaction. It is also desirable that the method give a quantitative estimate of the modulation in binding affinities.

A method suitable for high throughput screening is also needed, in order to screen a high number of componds.

The present invention provides a highly sensitive screening method and assay allowing the identification of compounds capable of modulating the intracellular interaction between two peptidic binding partners or capable of creating an interaction between these partners. The screening method of the invention is based on the sequential exposure of a candidate compound with each one of the binding partners. The method of the invention is preferably carried out in liquid medium.

According to the present invention, the candidate compound, which will be referred to as "C", being tested for its ability to modify the interaction between peptidic partners A and B, is allowed during a first stage to contact in a cell the peptidic partner A alone, i.e. in the absence of partner B.

This protocol allows the candidate compound to potentially interact with partner A, without the interference caused by the interaction between partners A and B. Subsequently, partner B is made available in the cell for binding with A. It is advantageously associated with a two-hybrid assay, preferably with luciferase as reporter gene.

A major application of the invention is the screening to identify small molecules that inhibit protein interactions. For this, distinguishing features of the assay lie in its ability to support high-throughput screening and to identify molecules that prevent the formation of protein complexes, as well as molecules that disrupt pre-existing complexes. This characteristic may increase significantly the hit rates of screening efforts aimed at identifying small molecule inhibitors of protein interactions.

Definitions in the context of the present invention:

By modulating an interaction between two peptidic partners is meant a change affecting any characteristic of the interaction, for example its affinity, its avidity, its duration or its resistance to proteolytic cleavage. Therefore, the modulation can be made in preventing, disrupting, stabilizing or modifying the interaction.

By creating an interaction between two peptidic partners is meant allowing a specific interaction to occur which did not previously occur; this can be made by generating de novo an interaction or restoring a lost interaction.

By exogenous compound with respect to a cell is meant a compound which comes from outside the cell or a compound, at least one precursor or component of which, comes from outside the cell. For example, any compound which is introduced into the cell is referred to as exogenous, irrespective of whether or not an identical compound is already present in the cell. A protein expressed from a transfected plasmid is also referred to as exogenous, in the context of the present invention.

Exogenous compounds may thus be heterologous to the cell in so far as no natural counterpart of these molecules exists in the cell, or may be homologous if the exogenous compound is essentially identical to a natural cell component.

By luciferase is meant an enzyme that catalyses the oxidation of substrate luciferins to yield non-reactive oxyluciferins and the release of photons of light [Greer and Szalay, 2002].

By peptidic binding partners is meant a set of two entities constituted essentially of proteins or peptides, exhibiting a biological interaction in a cell. For example, peptidic binding partners may be a protein and its receptor. Another example of a pair of binding partners is a pair of identical proteins known to dimerise. A peptidic binding partner also includes a protein complex, so peptidic binding partners also include a protein and a complex of proteins known to form a complex of higher order. The biological interaction which occurs between the binding partners is not usually a covalent peptide bond, it may involve ionic force, dipole-dipole interactions, etc...

The affinity between peptidic partners is generally characterized by dissociation constant at equilibrium (Kd) which is calculated by the concentration of complex created by the binding partners, divided by the product of the concentrations of each partner not engaged in a complex. A Kd generally ranges from $10^{-12}$ to $10^{-3}$ , and preferably from $10^{-9}$ to $10^{-6}$ (Dissociation constant Kd at equilibrium).

The interaction can also be indirect, i.e. it is achieved thanks to the presence of a third compound, for example, which stabilizes the interaction between the two binding partners.

By obligate interaction between two molecules is meant an interaction which is independent of the state of said molecules, for example independent of phosphorylation. On the contrary, a non-obligate interaction is conditional and depends on the state of the interacting molecules.

By highly sensitive reporter gene is meant that the reporter gene is activated as a result of weak interaction.

By highly sensitive reporter assay is meant that very small quantities of expressed reporter can be detected. This is the case when the concentration of yeast, transformed by the reporter gene, is very low. This assay can thus be performed with a very low number of transformed cells.

The present invention discloses an intracellular screening method which allows the identification of exogenous candidate compounds C capable of modulating the intracellular interaction between two peptidic binding partners A and B. The present method also allows the detection of exogenous candidate compounds C capable of creating an intracellular interaction between two peptidic molecules A and B which thus become peptidic binding partners. The intracellular interaction between the two components A and B is responsible for a specific given phenotype.

This method according to the invention is achieved inside a cell. The interaction between interaction partners may occur in the cytoplasm, in the nucleus of the cell, or in any other compartment of the cell.

According to the method of the invention, the following steps are carried out specifically in the following order:

- in a first step, the candidate compound C is allowed to contact the binding partner A, inside a cell, in the absence of the partner B,
- in a second step, the partner B is then made available for binding to partner A, and
- in a third step, the phenotype of said cell is detected.

By "specific given phenotype" is meant that the cell has a phenotypic trait which reflects the interaction of the binding partners. This phenotypic trait can be for example an acquired specific autotrophy, or the capacity to emit light upon excitation or in presence of a substrate, or the death of the cell. The phenotype can be visible (for example growth visible to the naked-eye) and/or detectable as a readout by any known measures, such as measure of the light emitted, the wavelength, the dosage of a compound, the intensity of a color, the optical density, etc...

This phenotype must be caused directly or indirectly by the interaction of the binding partners and be absent if such an interaction does not occur. According to the present invention, the given specific phenotype is the phenotype of the cell when the screening method is carried out without compound C.

This phenotype preferably discriminates between strong and weak interactions and between permanent and transient interactions. In a preferred embodiment, the intensity of the phenotype reflects the binding affinity of the partners A and B, for example it is proportional. The most preferred embodiment is a phenotype which is also capable of reflecting the changes intervening in the binding affinity of the partners, that is if the affinity decreases, due to the presence of compound C, the phenotype intensity also decreases.

According to the present method, the candidate compound C is given an advantage in being present in the cell with the first binding partner A before the second partner B is made available for also interacting with the partner A. Due to the sequential nature of the method of the invention, it must be noted that roles of partners A and B are not interchangeable. Indeed, exposure of partner A to compound C occurs before availability of partner B. The results thus obtained are not necessarily identical to those obtained when exposure of partner B to compound C occurs before availability of partner A. Accordingly it is an advantageous characteristic of the invention that the possible influence of the sequence of the steps can be determined.

According to the invention, the fist and second step are ideally separated by at least 30 minutes, for example by 1 hour or more, e.g. 2 or 3 hours.

The present invention allows the detection of compounds C capable of preventing the interaction between partners A and B even if compound C is not capable of disrupting the interaction once it is in place.

This is for example the case when the compound C occupies a site of partner A and thus hides it from partner B. It is also frequent that compound C, by its interaction with partner A triggers a conformational change in partner A. After this interaction between A and C has occurred, partner B is no longer a binding partner for A in its modified conformation.

The method of the invention allows the detection of such compounds C which can be classified as false negatives in classical screening methods where the compound to be tested is added <u>after</u>, or simultaneously with, formation of the interaction between the binding partners.

The present invention also allows the detection of compounds C which, without totally inhibiting the interaction between partners A and B, are capable of modifying the characteristics of such an interaction. It is thus possible to detect compounds C which decrease or increase the binding affinity or avidity of the interaction.

According to the screening method of the invention, compounds C capable of creating an interaction between partners A and B can also be detected. Such an interaction would not be possible without compound C. This situation is encountered when the interaction of partner A with compound C involves a conformational change of partner A allowing it to interact with partner B as is the case for protein binding ions or ATP or GTP and which have different binding partners in their free or bound state. The interaction thus created can be a totally novel interaction having no previously recognized existence or can be a previously lost interaction restored by the intervention of compound C. By lost interaction, is meant a known interaction involving the binding partners, which no longer occurs, for example because a mutation in one of the partners prevents the interaction or because an ion such as $Mg^{2+}$ is no longer present to permit the interaction.

According to the method of the invention, it is also possible to identify compounds C which are capable of converting a non-obligate interaction between partners A and B, to an obligate interaction.

According to the present invention, a compound C which provokes an interaction between partners A and B, which thus become binding partners, can also be detected. Such a compound C is for example an "adapter" which creates a link between partners A and B.

According to a preferred embodiment of the present invention, the given specific phenotype also decreases in intensity if the interaction ceases or is disrupted. The evolution of the interaction can thus be monitored. According to this embodiment, the present invention also concerns the detection of compounds C which stabilize the binding partners A and B. When the interaction is stabilized, the transcription of the reporter gene is more highly activated, thus generating a phenotype of greater intensity than when no stabilization is achieved. The integration over time of the level of transcription of the reporter gene will clearly reveal the stabilization of the interaction. It is therefore useful to discriminate between compounds C capable of stabilizing the interaction and those that are incapable of such an action.

According to a preferred embodiment of the present invention, the phenotype or phenotypic trait is the result of the expression of a reporter gene. This is a well known method for detecting phenomena occurring inside a cell. In the context of the invention, "reporter gene" is any gene, exogenous or endogenous, whose expression directly or indirectly reflects the presence or absence of a given interaction. Preferably, the reporter gene is an exogenous reporter gene, but it may also be endogenous.

The reporter gene is generally under the control of a promoter which triggers the expression of the reporter only when specific conditions are present. The reporter gene is generally integrated into the genome of the cell or is present on a plasmid inside the cell.

Certain reporter genes lead to the death of the cell when they are expressed. Assays based on reporter gene of this type are generally called "reverse assay". Preferred assays according to the invention are "forward assays". i.e. the given specific phenotype is not the death of the cell.

Well-known reporter genes are genes coding for nutritional autotrophy markers, such as HIS3 or LEU2. They allow yeast growth on selective media lacking the corresponding amino-acid. An alternative reporter gene is lacZ gene coding for β-galactosidase, which is widely used in different assays. It offers a convenient readout on solid media or supports containing the chromogenic substrate X-gal, on which the specific given phenotype is a blue color. This phenotype is also quantifiable by spectrophotometric liquid assays.

While the reporter genes featuring autotrophy markers are always integrated into the yeast genome (and exist therefore as a single copy per haploid yeast), lacZ reporter genes can be found either integrated into the genome, or carried by plasmids.

Other alternative reporter gene also envisaged in the present invention is a variant of the green fluorescent protein such as GFPuv, which has been used successfully to visualize phenotypes and to measure these phenotypes by flow cytometry. Such assays were shown to be highly quantitative in that the average fluorescence levels of yeast populations were proportional to the binding affinities of the different pairs of binding partners [Colas, 2000a].

Alternative reporter genes, more specifically used in mammalian cells, are genes for resistance to antibiotics such as hygromycin and neomycin, DHFR gene (dehydrofolate reductase) and surface markers, such as CD2 and H2K$^k$. Other reporter genes, commonly used in bacterial cells, are also genes for resistance to antibiotics, such as resistance to ampicillin, chloramphenicol, kanamycin and tetracycline.

In a preferred embodiment of the present invention, the binding partners A and B are components of a two-hybrid system.

According to this system, a first peptide, the bait, is produced as a fusion with a DNA-binding moiety and a second peptide, the prey, is produced as a fusion with a corresponding transcriptional activation domain. The interaction between first and second peptides brings together the DNA-binding moiety and the activation domain, thus reconstituting an active transcriptional activation domain. The reporter gene is under positive transcriptional control of the reconstituted transcriptional activation domain and its expression reflects the interaction between the prey and the bait.

In this embodiment, partner A can be either the bait or the prey, partner B being respectively the prey or the bait. With regard to DNA binding moiety and transcriptional activation domain, well-known constructions are used in the art. For example, such constructions make use of LexA, or GAL4, or other systems. Depending on the cell in which the two-hybrid assay is to be achieved, different constructions are available for one skilled in molecular biology.

According to the two-hybrid setting, partners A and B are expressed preferably from plasmids. Different plasmids can be constructed, by varying the ORI (origin of replication) of the plasmid, defining the copy number in a cell. However, it is also envisaged in the context of the present invention that genes coding for partners A and B, fused to their respective DNA-binding moiety and activation domain, are integrated into the genome of the cell.

An important aspect of the invention is the fact that B is provided to A only after A has been exposed to C. This may be achieved in a number of different ways, for example using any methods which allow a spatial or temporal regulation of the availability of B.

According to a particularly preferred embodiment of the present invention, the partner B is made available in the second step of the screening method by induction of its expression. This particular setting can be achieved by placing the gene coding for partner B under the control of an inducible promoter. If a two-hybrid system in yeast is used, which does not make use of the transcriptional module of GAL4, the inducible promoter can be the promoter GAL1. This promoter is repressed by glucose and can be induced by adding galactose.

In order to control exactly the time at which the partner B is made available for binding with the partner A, the culture conditions advantageously involve starving the yeast of glucose (e.g. by replacement with raffinose) a few hours before induction. This protocol ensures that no trace of glucose, which strongly represses the GAL1 promoter is present at the time of induction, thus allowing a rapid induction of the partner B expression by galactose.

The inducible character of the expression of partner B allows a fine control over the initiation of step (ii) in the screening method. This is particularly advantageous, because the duration of step (i) can be controlled and modified to determine its influence. It makes it also possible, when compound C is added from outside the cell, to allow this compound to reach the nucleus, thus increasing its effective concentration in nucleus, before inducing the expression of the second partner. This is particularly advantageous for compounds which penetrate slowly into the cell or nucleus.

It must however be noted that the induction system inherently involves a lag between the moment of induction and the moment of expression of partner B. Therefore, the necessary delay between intracellular availabilities of compound C and partner B can be achieved by exploiting an induction system for partner B.

The duration of this inherent lag may be from several minutes to 2 or 3 hours or more.

In the context of the present invention, it is also envisaged that both partners be under the control of inducible promoters. However, in this particular case, care should be taken to choose two different promoters in order to induce partners A and B in such a way that B becomes available only after contact between A and C, as required by the screening method of the invention.

According to the present invention, other ways may be envisaged to make available partner B at a delayed stage by reference to partner A. For example, it is possible to design a partner B which bears a thermo-sensitive mutation. In this case, at restrictive temperatures, partner B is not available for interacting with partner A, as it is present in the form of an incorrectly folded protein. It can thus be considered that partner B is not present, as "binding partner B" signifies a peptidic component capable of interacting with partner A. This peptidic component actually becomes a partner for the component A when the temperature is switched from a restrictive to a permissive temperature-Indeed, at permissive temperatures, the folding of partner B is suitable for interaction. By this switching, partner B is made available to partner A. Suitable thermo-sensitive mutations of many genes of organisms supporting classical genetic analysis are well known for one skilled in the art.

Another possible way of making partner B available at a later stage is achieved by sequestering said partner in a different cellular compartment, where partner A is not present. At the site of interaction between partner A and compound C, partner B is not present. Said partner B is then made available by allowing it to leave this compartment and to contact partner A and compound C, or by allowing it to enter the compartment where partner A is present. An example of such a situation is the sequestration of the partner B outside the nucleus, whereas partner A and compound C are allowed to interact in the nucleus of the cell. It is also envisaged that partner B is released from the sequestering compartment, for example by disruption of this compartment, or is allowed to enter a specific compartment containing partner A, e.g. inducing expression of a targeting signal etc...

According to the present invention, it is also envisaged that partner B is not available for interaction with partner A because a third component X is specifically bound to partner B and prevents any interaction with partner A. Partner B is made available at a later stage by making component X unavailable for the interaction with B. In such a case, care should be taken that the binding affinity of the interaction between B and X must be greater than the binding affinity of the interaction between A and B and/or the abundance of X is greater than that of B. If the condition is not respected, the presence of partner A will compete with component X therefore making partner B available for partner A at a stage which is not compatible with the features of the screening method of the invention.

Component X can be effectively made unavailable by adding a component Y which also interacts with X but with a stronger affinity than the interaction between X and partner B, or by switching the temperature if X bears a thermo-sensitive mutation.

An example of such an embodiment is a partner B comprising a FKBP domain (FK506 binding protein). This partner B can be made unavailable to partner A, by binding to a FKBP protein comprising a famesyl domain, via FK1012 (dimer of FK506), thus being sequestrated at the membrane due to the famesyl domain. Partner B can then be made available in the nucleus by adding an excess of FK506 (monomer) which will disrupt the previous association via the dimer FK1012 [Crabtree and Schreiber, 1996]. Another embodiment is given by the expression of a fusion protein comprising Cdk2 with a thermo-sensitive mutation and a famesyl domain, which is thus localised at the membrane. By switching the temperature from a permissive to a restrictive one, it is thus possible to make available in the nucleus a p21 protein for interaction with PCNA [Cayrol, 1997].

According to another embodiment of the present invention, partner B is made available at the desired time by introduction of this component inside the cell where the binding partner A and the compound C are already present. By direct introduction, is meant that the partner B can be introduced into a cell by any physical, chemical or biological means, for example with a micropipette, or can be added in the culture medium of the cell, or can be introduced by a calcium-phosphate method or electroporation.

It is also possible in the context of the present invention that the partner B is introduced in the form of DNA encoding said partner, for example by introduction of a plasmid bearing the gene coding for partner B with all the necessary elements ensuring its expression inside the cell after introduction.

Alternatively, partner B can also be introduced by fusion of the cell with another cell or with a liposome containing either partner B or DNA coding for it, or corresponding RNA. Another particularly advantageous way of introducing said partner B, when the screening method is achieved in yeast cells, is the mating between two types of cells. The first type contains partner A and compound C. The other type of cell contains either partner B or sequences capable of leading to the expression of partner B. When these two types of cells are allowed to mate [Finley and Brent, 1994], partner B is made available to partner A after compound C as required by the screening method of the invention.

The invention is not limited to these specific ways of making partner B available at a later stage than partner A. Other methods well known to the skilled person in the art are also encompassed in the present invention.

According to the present invention, compound C is an exogenous compound which may be peptidic or not.

In a specific embodiment of the invention, compound C is of non peptidic nature. Compound C is for example a small organic molecule, an inorganic compound, a metabolic substance, or compound C comprises DNA or RNA, or a hydrid duplex of RNA and DNA.

Examples of suitable peptidic compounds according to the present invention are conformationally-constrained peptides. In such a system, a randomly generated amino-acid sequence is covalently bound at both amino and carboxy termini, ensuring a constraint on the conformation of random sequence (see PCT application number WO96/02561). A population of compounds C, distinguished from each other by the sequence of the random peptide, can thus be tested according to the screening method of the invention.

According to a preferred embodiment of the screening method of the invention, at least one of partners A and B is conformationally-constrained, compound C being either conformationally-constrained or not.

In a preferred embodiment of the present invention, at least one of binding partners A and B is a protein complex, or both are protein complexes. A complex of proteins contains at least 2 proteins, which can be identical or different, but may contain 3, 4, 5 or even more proteins, different or identical. Suitable examples of binding partner are the complex Cdk-Cydin which interacts with p27 and the complex E6+E6-AP which interacts with p53.

According to the present invention, the screening method is preferably carried out in a cell which is a yeast cell, for example a cell of *S. cerevisiae.* Alternatively, bacterial cells such as E. coli cells, or mammalian cells such as CHO cells, can be used to carry out the method of the invention. Human cells, for example immortalized cells like HeLa can also be used according to the present invention.

As mentioned previously, in order to carry out the screening method of the invention, care should be taken as to the choice of the reporter gene, when such a reporter gene is used for obtaining a detectable phenotype. The well documented lacZ reporters or GFP-*derived* reporters can be used in the context of the present screening method.

Advantageously, the reporter gene is chosen in order to render the method rapid, sensitive, with a wide dynamic range and capable of quantitatively estimating binding affinities.

In order to obtain such a method, a particularly preferred reporter gene is a luciferase gene, for example the *luc* gene which comes from a firefly species *(Photinus pyralis)* or the *ruc* gene, which comes from a sea pansy (*Renilla reniformis).* These reporter genes have proven to have considerable qualities in terms of sensitivity and wide dynamic range of their detection assays (see experimental part of the present invention).

These reporter genes are preferred due to the distinguishing features of luciferases in comparison to other reporters commonly used. In contrast with β-galactosidase which is a tetrameric enzyme, both luciferases are monomeric, and in contrast with GFPs, neither of them require a long folding process. Therefore, both enzymes can function immediately upon translation of their coding messengers. This also opens the possibility of destabilizing the stability of *luc* and *ruc* proteins to increase the sensitivity of the assay, for example by inserting PEST sequence [Rechsteiner, 1996].

However, β-galactosidase reporter is also suitable in the context of the present invention since luminogenic substrates have been developed to enhance the sensitivity of detection of β-galactosidase activity. Moreover, it has been reported recently that two-hybrid β-galactosidase signals were successfully detected from 80µl saturated yeast suspensions delivered into 96 well plates, with an induction time of 4 hours [Clark, 2003].

Two GFP variants (YFP and CFP) may also be used as reporter genes. However, in this case, two-hybrid fluorescent phenotypes must be preferably detected upon excitation with a laser beam (confocal microscope and flow cytometer).

Moreover, brighter variants than YFP and CFP, like GFPuv can also be used. Indeed, the GFP reporters appear to be less sensitive than the reporter mentioned above. One reason contributing to the lower sensitivity of the GFP reporters is that, in contrast with β-galactosidase and luciferases, there is no signal amplification with GFPs. Another reason is the autofluorescence of yeast and the absorption of fluorescence by the culture medium.

The screening method can also be adapted to be carried out in high throughput screening systems (HTS). In order to carry out the method in this context, the phenotypic trait is preferably the expression of a reporter gene. As HTS implies several screens achieved simultaneously in 96-well or 384-well plates, the sample volumes must be small and the reporter system (i.e. the combination of reporter gene and reporter system) chosen to be sensitive enough to be detected even if very small volumes are used. A highly sensitive reporter system is therefore required in order to carry out the invention in the context of HTS. Assays compatible with the use in HTS are described in the experimental part of the present application.

The screening method of the invention allows the detection compounds C which are capable of modulating or creating an interaction between partners A and B. Optionally, after identification that a specific compound C is capable of modifying the phenotype, said compound is retrieved and isolated. A further optional step is the synthesis of analogues of said compound C for optimisation of the modulatory activities. In an additional further step, this compound is duplicated, copied or manufactured on a scale sufficiently large for further use in screening methods or therapeutic or diagnostic uses. Such a compound can then be used to modulate the interaction between partners A and B in cells wherein such an action is needed. Specifically, such a compound C can be used in therapeutic or prophylactic methods to treat or prevent diseases or disorders.

In a preferred embodiment of the present screening method, compound C is not only tested for its ability to modulate the interaction between a first pair of binding partners A and B, but is also tested for its ability to modulate the interaction between a second pair of binding partners A' and B'. By this double-screen, it is possible to detect exogenous candidate compounds capable of modulating the intracellular interaction between the two peptidic binding partners A and B and being incapable of modulating the interaction between the peptidic binding partners A' and B'. According to this double-screen, it is also possible to detect the capacity of compound C to create an interaction between partners A and B and also its capacity or inability to create an interaction between partners A' and B'.

It is indeed a major advantage in the field to detect compounds capable of modulating an interaction in a specific manner rather than through non-specific toxicity, or through an interference with the readout of the 2 hybrid phenotype, or through a pathway common to several binding pairs.

According to this embodiment of the invention, the interaction between partners A and B is responsible for a specific given phenotype P and the interaction between partners A' and B' is also responsible for a specific given phenotype P'. These phenotypes are detected after the following sequence of steps:

There is a first stage wherein the candidate compound is allowed to contact the binding partner A, inside a cell, in the absence of the partner B, and is also allowed to contact the binding partner A' inside a cell, in the absence of partner B' and a second stage wherein the partners B and B' are then made available for binding with their respective partners A and A'.

By conducting this specific embodiment of the screening method of the invention, it is thus possible to discriminate between compounds C which modify the phenotype P by a specific action on the interaction between A and B and compounds C which modify the phenotype P by a non-specific pathway. Indeed, in the first case, if the action of compound C is specific to the interaction between partners A and B, the phenotype P' is not affected by the compound C and thus the phenotype P is the sole affected phenotype.

In the second case, compound C is for example a toxic compound and in this case addition of compound C affects phenotypes P and P'.

It is thus possible to discriminate between compounds C modulating specifically an interaction and compounds C modulating said interaction due to a general toxic effect.

Should the peptide A have two different partners B and B', it is also possible to detect compounds C affecting specifically the interaction between A and B and which do not affect the interaction between A and B'. According to this situation, peptides A and A' are identical.

Alternatively, it is also possible to detect compounds C which actually modify interactions between A and B and between A' and B'. According to the previously described situation, it is therefore possible to detect compounds C which simultaneously affect the interaction of peptide A with both its binding partners B and B'.

It is also evident that, if partner B has two distinct binding partners A and A', according to the double-screen embodiment of the invention, it is possible to screen compounds C against the interaction between A and B and between A' and B, where B and B' are identical.

Moreover, as indicated above, the role of partners A and B are not interchangeable according to the invention, it is thus possible to carry out the double screen embodiment with partners A and B and with partners B and A. In this particular case, we have A' identical to B and B' identical to A. This specific embodiment is particularly advantageous to test whether the modulation of compound C on the interaction between partner A and B is due to a direct interaction of C with A or due to a interaction of C with B.

Indeed, according to the screening method, an advantage is given to the interaction between one of the binding partners (called A) and the tested compound C, over the interaction between A and B, and over the potential interaction of B (the second binding partner) with C. Therefore, comparison of the results obtained when roles of A and B are exchanged gives informative data on the preferential interaction of C with A or with B. This enables the discrimination between compounds C capable of modulating the interaction between partners A and B by direct interaction on partner A and compounds C capable of the same modulation by direct interaction on partner B.

According to a specific embodiment of the double screen embodiment, interactions between partners A and B and between partners A' and B' occur in the same cell. In this particular case, it is evident that phenotypes P and P' must be different, in order to detect each one.

Alternatively, the interaction between partners A and B and the interaction between, A' and B' can occur in two different cells. In this case, the phenotypes P and P' are not necessarily different and can be identical. The phenotypes P and P' are preferably distinct phenotypes. This feature allows the mixing of the two different types of cells in the same sample. These cells can thus be mixed in the same vial or in the same well; the two distinct phenotypes allowing the specific detection of the modulation of compound C on the interaction between partners A and B in the first type of cells and on the interaction between partners A' and B' in the second type of cell.

In this specific embodiment, the mixing of the cells allows the simultaneous implementation of the double screen on the two types of cells. This specific embodiment, called duplex, presents many advantages. This indeed allows, in the same well, to screen a compound C on a interaction between partners A and B and simultaneously to detect the effect of compound C on another interaction, for example a negative control. This is advantageous because it saves money and time and because it ensures that the conditions are identical for the test interaction and the control interaction by preventing the variation between wells. Two pieces of information are obtained from a single well.

This embodiment of the screening method of the invention is ideally suited for high throughput screening of small molecule modulators of protein interactions.

According to a preferred embodiment of the present invention, the different phenotypes P and P' arise from the expression of different reporter genes. It is particularly advantageous that said reporter genes are two different luciferase genes, for example *luc* from *Photinus pyralis* and *ruc* from *Renilla reniformis.*

Alternatively, it is also envisaged according to the present invention to use different variants of Green Fluorescent Protein. Indeed, this protein is known for its fluorescent properties and variants have been elaborated with increased fluorescence intensity and different wavelengths in emission. Such variants are for example the following reporter genes: YFP and CFP.

It is clear that this embodiment of the screening method of the invention is not limited to a double screen and other interacting partners can also be tested in a parallel manner. For example, the action of compound C may also be assayed for its ability (or inability) to modulate the interaction between the binding partners A" and B". The interaction between partners A" and B" is also detected by a given specific phenotype P", this phenotype being identical or different from the phenotypes P and P'. Should this third interaction occur in the same cell as interaction between A and B, or between A' and B', the phenotype P" must be different from the phenotype P or P' respectively. Other additional interactions can also be tested in the same manner.

According to a preferred embodiment of the invention, the three or more interactions occur in three or more different cells and these cells are preferably mixed together in the same sample provided the three or more phenotypes are distinct form each other. This assay is thus called multiplex.

Binding partners according to the present invention are advantageously: TGFβ-R and FKBP12; TGFβ-R and FK506; FKBP12 and FK506; TR-α and NCoR; Cdks (including Cdk2), Bcl2, Bcl2-like proteins, RAS, RasGAP and IAPs, and their binding partners, including aptamers.

The screening methods of the invention are preferably carried out in a liquid medium.

Legend of the figures:

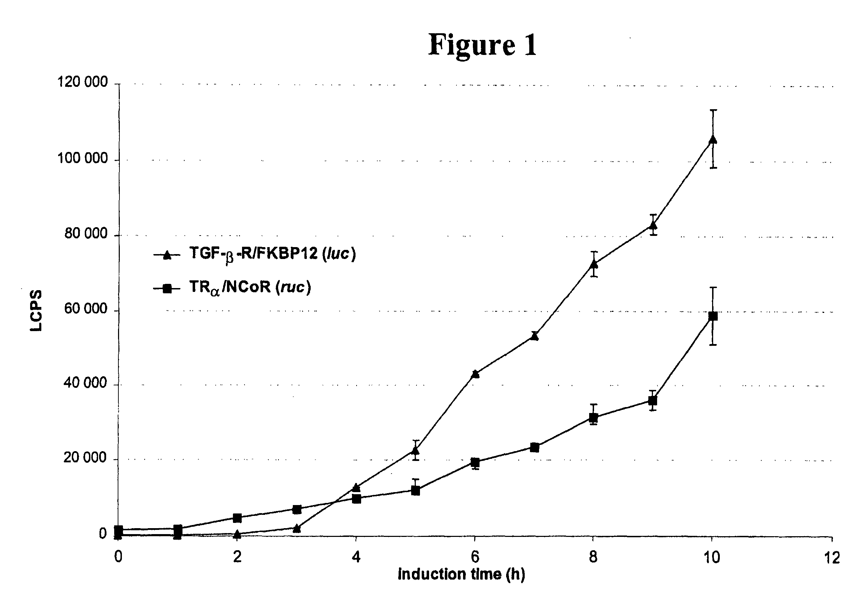

**Figure 1:** Kinetics of two-hybrid luminescent signals

TB50α yeast were co-transformed with pHB2-*luc* and plasmids directing the expression of lexA-TGFβ-R and B42-FKBP12, or with pHB2-*ruc* and plasmids directing the expression of LexA-TR-α and B42-NCoR. Yeast were grown from frozen transformants, and prey expression was induced for different times (0 to 10h). Luminescence was measured in duplicates.

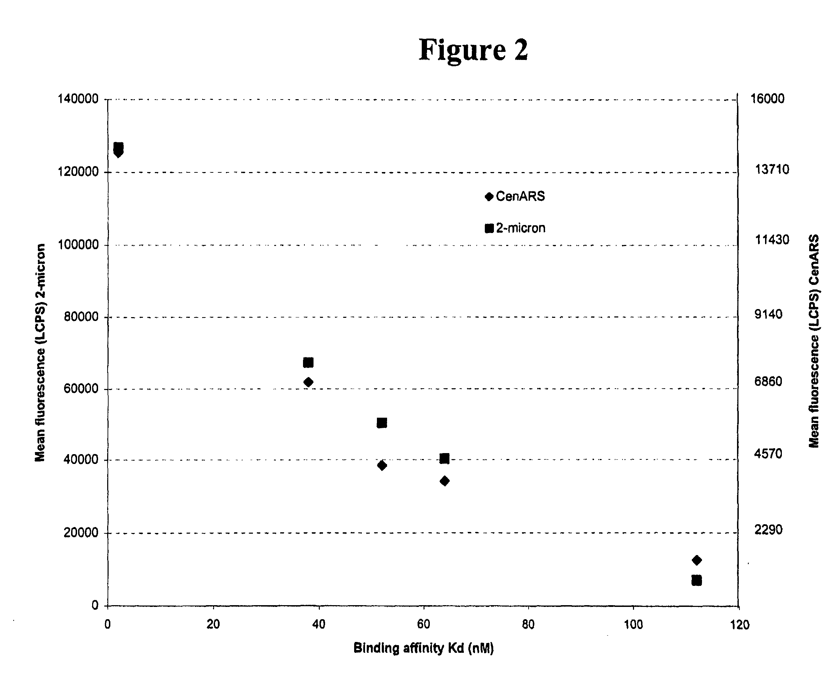

**Figure 2:** Luminescent two-hybrid signals and binding affinities

The TB50α yeast strain was co-transformed with pHB1-*luc* or pHB2-*luc*, a plasmid directing the expression of LexA-Cdk2 and different plasmids directing the expression of Cdk2 aptamers fused to an activation domain. Yeast were grown from colonies of transformants and the expression of peptide aptamers was induced for 4 hours, in 4 different wells per interaction.

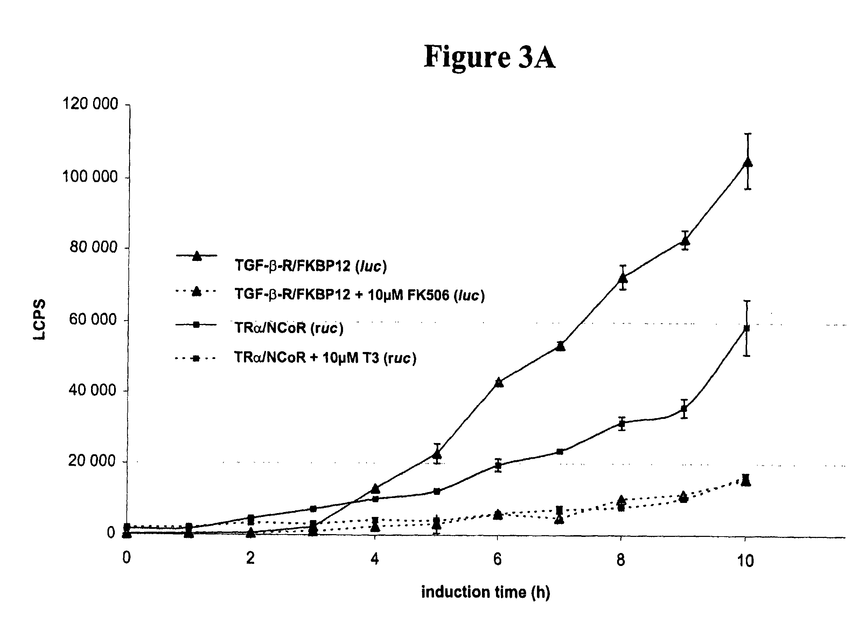

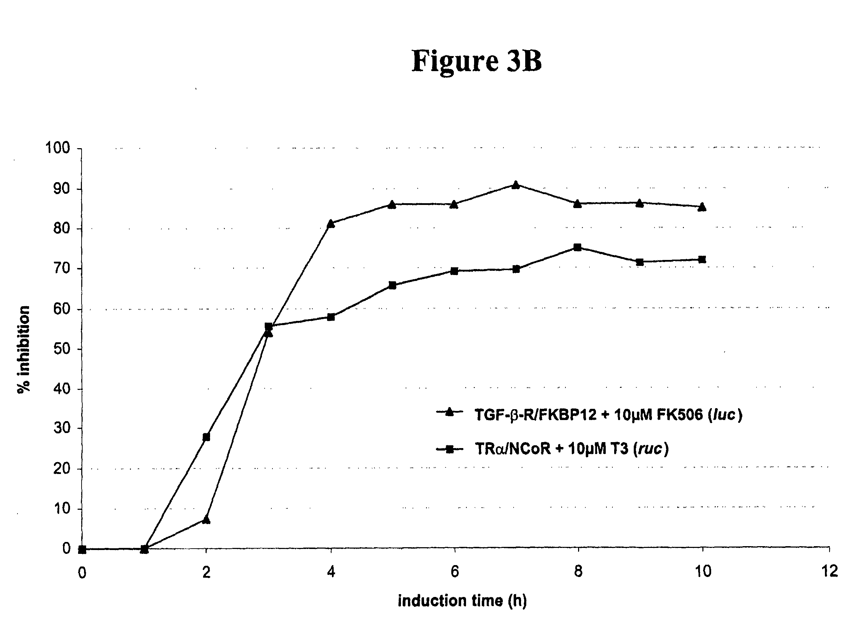

**Figure 3** : Detection of inhibition of protein interaction by small molecules

The experiment was described in Figure 1. Yeast were grown from frozen transformants, and prey expression was induced for different times (0 to 10h) in presence of DMSO (shown in Figure 1), 10µM FK506, or 10µM T3.

(A): Two-hybrid luminescent signals according to induction time, with and without inhibitors of protein interactions. Luminescence was measured in duplicates.

(B): Inhibition of protein interaction by FK506 and T3 according to induction time. The values are obtained through the following formula:

$$\% \text{ inhibition} = 100 - (\text{signal with inhibitor/signal without inhibitor}) \times 100.$$

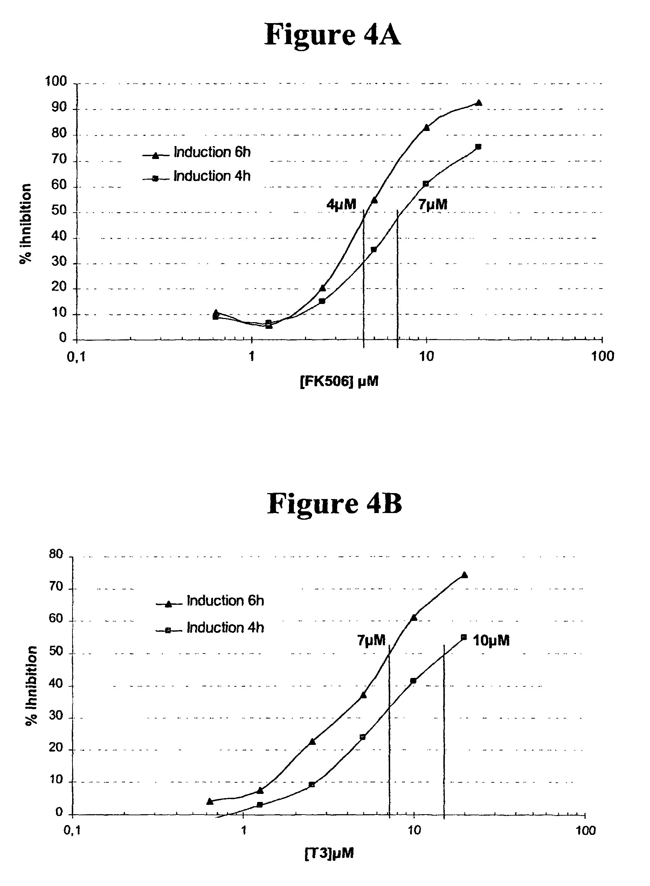

**Figure 4 :** Dose-response experiments of inhibition of protein interactions

Yeast were transformed as described in Figure 1. They were grown from frozen transformants. Two-hybrid assays were performed with induction times of 4h and 6h, in presence of various concentrations of FK506 (A) or of T3 (B). IC50 are indicated on the curves.

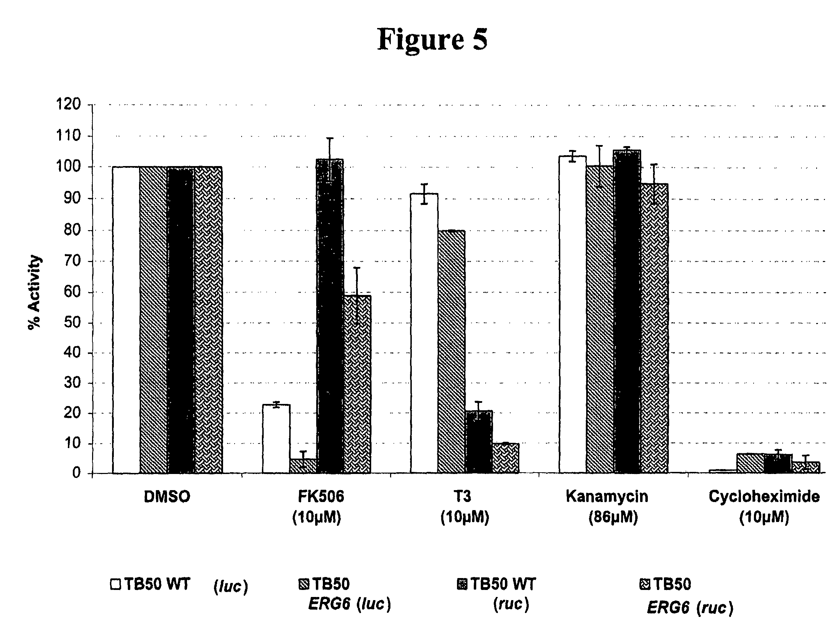

**Figure 5** : Selective detection of inhibition of protein interaction in a dual two-hybrid assay

Yeast strains TB50 WT and TB50 ERG6 were transformed as described in Figure 1. They were grown from frozen transformants. For each strain, the two yeast cultures containing the two protein interactions were mixed together and delivered into a microwell plate. Two-hybrid assays were performed with induction times of 5h, in presence of DMSO, FK506, T3, Kanamycin or Cycloheximide at the indicated concentrations. Experiments were performed in duplicates. The values are normalized with the signals obtained in presence of DMSO.

EXAMPLES:

The inventors have made use of an existing two-hybrid system, the interaction trap, developed in the laboratory of Roger Brent [see Golemis, 1997 for review]. However, new reporter genes and new protocols have been further developed to endow this assay with the qualities described in the present invention.

Choice of a sensitive reporter system:

In the past, a wide variety of reporter genes have been implemented in the various versions of the two-hybrid technology [see Vidal, 1999b for a review]. To enable selections from large populations of yeast, nutritional autotrophy markers such as HIS3 or LEU2 are always used. They allow yeast growth on selective media lacking the corresponding aminoacid. The *lacZ* gene, coding for β-galactosidase, is also widely used in the different systems. It offers a convenient readout on solid media or supports containing the chromogenic substrate X-gal, on which the two-hybrid phenotype is a blue color [Serebriiskii, 2000]. While the reporter genes featuring autotrophy markers are always integrated into the yeast genome (and exist therefore as a single copy per haploid yeast), *lacZ* reporter genes can be found either integrated into the genome, or carried by plasmids harboring two kinds of replication origins. The CEN/ARS system contains centromeric sequences that maintain low copy number of plasmids (1 or 2) in a given yeast and sequences that allow autonomous replication. The 2-micron replication origin gives rise to a high copy number of plasmids (20 to 50).

Although these reporter genes have proved very robust for two-hybrid screening of various kinds of DNA libraries (cDNA, combinatorial DNA, etc.), they offer readouts which are not optimal to perform quantitative two-hybrid assays. It is possible to quantify β-galactosidase activity through spectrophotometric assays, shown to be somewhat quantitative in that the measured values correlate to the binding affinities of the interacting proteins that are analyzed. However, these *lacZ* reporters show great limitations in their sensitivity level and their dynamic range. Furthermore, results can vary a lot according to the chosen protocol, as revelation of β-galactosidase activity is very sensitive to a number of variables [Serebriiskii, 2000].

The use of alternative reporter genes has also been explored in the past. A variant of the green fluorescent protein (GFPuv) has been used successfully to visualize two-hybrid interaction phenotypes and to measure these phenotypes by flow cytometry [Colas, 2000a]. Such assays were shown to be very quantitative in that the average fluorescence levels of yeast populations were proportional to the binding affinities of the different pairs of interacting proteins [Colas, 2000a].

Luciferase reporter genes:

Luciferases are enzymes that emit light in presence of oxygen and a luciferin substrate. Two eukaryotic luciferases are used widely in modem Biology, mostly as reporters to measure and visualize gene expression in mammalian cells. The first one, *luc,* comes from a firefly species *(Photinus pyralis).* The second one, *ruc,* has been used more recently and comes from a sea panzy *(Renilla reniformis).* These reporter genes are well appreciated for the sensitivity and the wide dynamic range of their detection assays. Recent technical advances have extended significantly the lifetime of the signals and have allowed to detect light emitted by both luciferases sequentially, in a single vial or well. The use of a luciferase reporter gene in the yeast *Saccharomyces* cerevesiae has been already documented, and also the use of *luc* and *ruc* as reporter genes in two-hybrid assays [Wang, 2002].

The use of luminescent reporter genes tremendously improves the existing two-hybrid assays, mostly in terms of sensitivity and dynamic range of the detection of protein-protein interactions. Such improvements open the way to, or at least facilitate significantly, certain applications of yeast two-hybrid assays, and is therefore particularly suitable for the screening method of the present invention.

Materials and methods

Construction of *luc* and *ruc* two-hybrid reporter genes

*CEN/ARS reporter plasmids:* The inventors started from pRS316, a yeast plasmid that carries a Ura3 marker and a CEN/ARS replication origin [Sikorski, 1989]. They amplified the LexAop promoter region of pSH18-34 [Golemis, 1997] using the oligonucleotides

5'-**TATATACCGCGGCATATCCATATCTAATCTTACC-3'** ;

and

5'-**TAAGACGCCGGCGATATCAAAAAAGAGGAACTGC-3'**.

which contained, respectively, a SacII and a NotI site. The PCR product was ligated into *SacI/NotI*-cut pRS316 to create pRS316-LexAop.

The ADH terminator of pJG4-5 [Golemis, 1997] was amplified using the oligonucleotides

5'- **CCGACTCGAGAAGCTTTGGACTTCTTCGCC-3'**

and

5'- **GGGGTACCGTGTGGAAGAACGATTACAACAGG-3'**,

which contained, respectively, a *XhoI* and a *KpnI* site. The PCR product was ligated into *XhoI/KpnI*-cut pRS316-LexAop to create pHB1.

The firefly luciferase cDNA from pGEMAE-luc (Promega) was amplified using the oligonucleotides

5'- **ATAAGAATGCGGCCGCATGGAAGACGCCAA AAACATAAAGAAAGG-3'**

and

5'- **AAGAAGTCCAAAGCTTCTCGAGTTACAATTTGGACTTTCCGCCCTTCTTG-3'**,

which contained, respectively, a *NotI* and a *XhoI* site. The PCR product was ligated into *NotI/XhoI*-cut pHB1 to create pHB1-luc.

The Renilla luciferase cDNA from pRL-SV40 (Promega) was amplified using the oligonucleotides 5'ATAAGAATGCGGCCGCATGACTTCGAAAGTTTATGATCC-3' and
5'- AAGAAGTCCAAAGCTTCTCGAGTTATTGTTCATTTTTGAGAACTCGC TCAACG-3'
which contained, respectively, a *NotI* and a *XhoI* site. The PCR product was ligated into *NotI/XhoI-cut* pHB1 to create pHB1-*ruc*.

*2-micron reporter plasmids:* The inventors started from Yeplac195, a yeast plasmid that carries a Ura3 marker and a 2-micron replication origin. Plasmids pHB1-*luc* and pHB1-*ruc* were digested with Sacl and *Kpnl* and ligated the inserts into Sacl/Kpnl-cut Yeplac195 to create pHB2-*luc* and pHB2-*ruc* respectively.

Construction of baits and preys

In the present yeast interaction trap, baits are proteins fused to LexA (partner A) and preys are proteins fused to the B42 activation domain (partner B) [Golemis, 1997]. The plasmids directing the expression of LexA-TGFβ-R and B42-FKBP12 were obtained from Tongwen Wang [Wang, 1994]. A pAS2-1 plasmid was obtained from Patrick Ravel-Chapuis [Bai, 1997] containing the cDNA of TR-α1. This plasmid was digested by *BamHI* and the insert cloned into BamHI-cut pEG202 [Golemis, 1997]. A pVP16 plasmid was obtained from Patrick Ravel-Chapuis [Vojtek, 1997] containing a NcoR partial mouse cDNA (Genbank accession number NM_011308, nucleotides 1410 to 7241). This plasmid was digested by *Eco*RI and the insert cloned into *Eco*RI-cut pJG4-5 [Golemis, 1997].

In order to control the time at which the prey is made available for binding with the bait, the prey in under the control of an inducible promoter, specifically GAL1. This promoter is induced by addition of galactose in the culture medium. Inversely, this promoter is repressed in presence of glucose. Therefore, the culture conditions have been performed in order to replace glucose by raffinose a few hours before induction. This protocol ensures that no trace of glucose, which strongly represses the GAL1 promoter is present at the time of induction, thus allowing a rapid induction of the prey expression by galactose.

Because *ERG6 TRP1* strains are not viable, there was a need for a prey expression vector other than pJG4-5 which contains a TRP1 marker. The inventors built by homologous recombination in yeast pLP3, a prey expression vector bearing a LEU2 marker. The fusion sequence NLS-B42-HA from pJG4-5 was amplified using the oligonucleotides 5'- CCACTTTAACTAATACTTTCAACATTTTCGG-3' and 5'-CAAGCCGACAACCTTGATTGG-3'. EGY48 was co-transformed [Golemis, 1997] (see below) with the resulting PCR product and *Eco*RI/*Xhol*--cut pBC103. Transformants on SD L- agar plates were selected and pLP3 was recovered by yeast DNA mini-preparation [Golemis, 1997]. The inventors introduced FKBP12 and NcoR cDNAs into pLP3 by homologous recombination in yeast. Both cDNAs were amplified from pJG4-5 using the same oligonucleotides used to build pLP3. EGY48 was co-transformed with each PCR product and EcoRI/Xhol-cut pLP3. Transformants were selected and the plasmids were recovered as described above.

Yeast manipulations

Transformation and storage

The strain used for the interaction assays is TB50 (Mat *leu2-3 ura3-52 trp1 his3 rme 1HMLa).* Yeast transformation was performed as described [Gietz, 1992]. To freeze yeast transformants, cultures were grown overnight in UHW 2% glucose liquid medium. Glycerol was added to a final concentration of 20%. The mixtures were aliquoted and frozen at -80°C.

Cultures from colonies of transformants

A few yeast colonies transformed with a bait, a prey and a reporter plasmid were picked from SD U-H-W- agar plates and used to inoculate a SD U-H-W- 2% glucose liquid culture, grown for 5 to 6h at 30°C under agitation. Yeast were then collected by centrifugation and resuspended in SD U-H-W- 1% raffinose/1% glycerol liquid medium, aiming at a density of OD600 = 0.2. Yeast were grown overnight at 30°C under agitation. The OD600 were then adjusted at 0.7 in the same medium.

Cultures from frozen transformants

Aliquots of frozen transformants were thawed and used to inoculate a SD U-H-W- 1 % raffinose/ 1% glycerol liquid culture (1 volume of frozen transformants for 15 volumes of medium, which corresponds to an approximate OD600 of 0.35). Yeast were grown for 23h at 30°C under agitation. Yeast density was then adjusted at an OD600 of 0.8.

This yeast culture ensures that no trace of glucose is present before induction.

Targeted disruption of *ERG6*

An erg6 deletion cassette was amplified from the genomic DNA of a erg6::TRP1 strain (STRg6, Stéphane Bach et al 2003 Nature Biotechnology, 21, 1075-1081) using the oligonucleotides 5'-CGGGTGTTTTCTCCTATCC-3' and 5'-TGTTTGTAAGGCCTGCTAGC-3'. The genomic DNA was a gift from Marc Blondel.

TB50a was transformed with the PCR product and transformants were then selected on SD W- 2% glucose medium. Several transformants were picked and the disruption of the erg6 gene confirmed by PCR using the above-mentioned oligonucleotides. The genomic DNA of one of the transformants was extracted and the disruption was confirmed by sequencing.

Two-hybrid interaction assays

20µl of yeast cultures were delivered into 96 well half area white plates (Coming-Costar, ref. 3693). When applicable, 2µl of either DMSO 10% or 10µM of small molecules were added. 2µl of Galactose 20% were added to the wells to induce the expression of the preys. When two different yeast cultures were mixed in a single well, 10µl of each culture were used, to reach a total of 20µl. Plates were incubated at room temperature and left non-agitated for various times.

To reveal the activity of both luciferases, a Dual-Glo™ luciferase assay system was used (Promega, ref. E2920) according to the instructions of the manufacturer. Briefly, to lyse the yeast and reveal the activity of firefly luciferase, 25µl of reagent was added and the plates were incubated for another 40 to 60min at room temperature, without agitation, before being read in a luminometer. To reveal the activity of Renilla luciferase, 25µl of "stop&glo reagent" were added to the wells and the plates were incubated for another 60min at room temperature, without agitation, before a second reading. Luminescence was measured by a Trilux (Perkin Elmer) setting the integration at 1sec/well. Values are given in LCPS (1LCPS = 100 counts per sec).

Results

Demonstration of sensitivity of the luminescent assay

From the existing pSH18-34 *lacZ* reporter plasmid, the inventors built 8 LexAop-*luc* and -*ruc* reporter genes carried by plasmids bearing either a CEN/ARS or a 2-micron replication origin. Because luciferase reporter gene assays are reputed for their great sensitivity, the inventors confirmed that it was possible to detect two-hybrid signals from low amounts of yeast, only a few hours after inducing the expression of the preys. To this end, two protein interactions were used that can be scored through yeast two-hybrid assays: The interaction between the type I receptor for TGF-$\beta$ R1C and the immunophilin FKBP-12 [Wang, 1994] and the interaction between the thyroid hormone receptor TR-$\alpha$ and the transcriptional co-repressor NCoR [Seol, 1996].

In order to achieve a rapid induction of prey expression by galactose, yeast cultures were performed so as to ensure that no trace of glucose, which strongly represses the GAL1 promoter, would be present at the time of induction (see Methods). As shown in Figure 1, 20µl of yeast suspensions produced luminescent signals that were readily detected, as soon as 3 hours after inducing the expression of the preys (below referred to as "induction time"). The two-hybrid signals produced by both protein interactions increased steadily with time and did not reach a plateau after 10h of induction time.

Similar results were obtained for both protein interactions using either *luc* or *ruc* reporters (data not shown).

Demonstration of quantitative nature of luminescent assays

In order to test the luminescent reporter genes in two-hybrid interaction assays and to determine whether they yield signals that correlate with the binding affinities of the interacting pairs of proteins, the inventors made use of an existing set of peptide aptamers (conformationally-constrained random peptides) that were selected for their ability to bind human cyclin-dependant kinase 2 (Cdk2) [Colas, 1996]. Peptide aptamers are combinatorial protein reagents that consist in a constant protein scaffold displaying a doubly-constrained peptide loop of random sequence [Colas, 2000b] for a review. The dissociation constants at equilibrium (Kd) between a set of peptide aptamers and Cdk2 were measured by surface plasmon resonance (SPR) experiments [Colas, 1996]. A mutated variant of one of the aptamers (10M) was obtained through in vitro evolution experiments and its Kd was also measured by SPR [Colas, 2000a].

The luminescence produced from the *luc* reporter on a CEN/ARS and a 2-micron plasmid (Table 1) were measured. These results show that the luciferase reporter gene yields signals that evolve in the same manner as the binding affinities of the two-hybrid interacting partners (see figure 2). Whereas the correlation observed using a GFPuv reporter was logarithmic [Colas, 2000a], the present equation from the *luc* reporter data seems more likely to come close to a proportional ratio. The fact that correlations were observed with both *luc* reporter plasmids (CEN/ARS and 2 micron replication origins) gives a first demonstration of the excellent dynamic range of the system. Similar results were obtained using the *ruc* reporter.

Table 1:

| Luminescent two-hybrid signals and binding affinities | | | | | |
|---|---|---|---|---|---|
| | | *CenARS* | | 2μ | |
| Aptamer | $K_d$ (nM) | Mean Luminescence (LCPS) | Standard deviation | Mean Luminescence (LCPS) | Standard deviation |
| 3 | 112 | 1449 | 81 | 7148 | 53 |
| 2 | 64 | 3896 | 118 | 40266 | 4501 |
| 5 | 52 | 4393 | 62 | 50278 | 477 |
| 8 | 38 | 7074 | 197 | 67387 | 2253 |
| 10M | 2 | 14326 | 994 | 126859 | 2438 |

The TB50α yeast strain was co-transformed with pHB1-*luc* or pHB2-*luc*, a plasmid directing the expression of LexA-Cdk2 and different plasmids directing the expression of Cdk2 aptamers fused to an activation domain. Yeast were grown from colonies of transformants and the expression of peptide aptamers was induced for 4 hours, in 4 different wells per interaction.

Detection of inhibition of protein interactions by small molecules

Because of its extreme sensitivity, the above-described luminescent two-hybrid assay can be performed in very low volumes, compatible with the use of 384 well plates. This format has become the favorite standard for high-throughput screening (HTS) of small molecule drugs. To determine whether the above described two-hybrid assay could be used for HTS, the inventors tested the capacity of said assay to detect the inhibition of protein interactions by small molecules in microwells. The interaction between the type I receptor for TGF-β and FKBP-12 is inhibited by the macrolide FK506 [Wang, 1994]; whereas the interaction between TR-α and NCoR is inhibited by the T3 hormone [Tagami, 1997].
The experiment shown in Figure 1 was performed in the presence of FK506 or T3 (Figure 3 A). In both cases, the signals were strongly inhibited by the small molecules disrupting their cognate protein interactions. FK506 and T3 produced over 90% and 70% inhibition of two-hybrid signals, respectively. Maximum inhibition was obtained after 4h of induction time for FK506, and after 6h of induction time for T3 (Figure 3 B).
The half-inhibitory concentrations (IC50) of both molecules were determined for induction times of 4h and 6h. The IC50 of FK506 was 7μM and 4μM (Figure 4A), and the IC50 of T3 was 10μM and 7μM (Figure 4B), respectively.

Inducible expression of preys confers increased sensitivity of detection of inhibition of protein interaction

In contrast with the interaction trap used in the present invention, baits (partner A) and preys (partner B) are expressed constitutively in most other yeast two-hybrid assays [Vidal, 1996]. In all experiments described above, the inventors have induced the expression of the preys (partner B) after adding the small molecules (compound C): Theoretically, this scheme should confer a strong advantage to the small molecules, which can reach and bind their protein target (partner A) before the protein complex between partners A and B is formed.
The inventors have shown that this advantage produces better inhibitions of protein interactions than that observed when baits and preys are constitutively expressed and thus when protein complexes are formed prior to the addition of small molecules.
Table 2 shows that for both protein interactions, the inhibition of the two-hybrid signals was stronger when the expression of the prey was induced after the addition of small molecule than when it was induced the day before. This latter experimental scheme reproduced two-hybrid settings in which the expression of both interacting partners is constitutive.

Table 2:

| Detection of inhibition of protein interaction in the context of a constitutive vs inducible prey expression | | | | |
|---|---|---|---|---|
| | Addition of compound C 23h after induction of prey (partner B) | | Addition of compound C before induction of prey (partner B) | |
| | % Inhibition 10μM | IC50 (μM) | % Inhibition 10μM | IC50 (μm) |
| TGF-β-R/FKBP12 (FK506) | 46% | 10 | 80% | 5 |

Table 2: (continued)

| Detection of inhibition of protein interaction in the context of a constitutive vs inducible prey expression | | | | |
|---|---|---|---|---|
| | Addition of compound C 23h after induction of prey (partner B) | | Addition of compound C before induction of prey (partner B) | |
| | % Inhibition 10µM | IC50 (µM) | % Inhibition 10µM | IC50 (µm) |
| TRα/NCoR (T3) | 58% | 5 | 74% | 5 |

Yeast strain TB50 WT was transformed as described in Figure 1. Transformants were grown from frozen transformants. The cultures were then split in two. In one case (left column), yeast were delivered into a multi-well plate and incubated in the presence of galactose to induce expression of the prey for 23h. Different concentrations of small molecules were then added to the wells and the plates were read 5h later. In the second case (right column), yeast were processed as described above (i.e. addition of small molecules and galactose, plates read after 5h induction time).

The duplex experiments:

The introduction of a second luciferase reporter gene (*ruc*) has provided a most convenient control to studies aiming at detecting a down-regulation of gene expression.

Indeed, while a decrease in gene expression can be exerted by a specific regulatory mechanism or by the specific action of a molecule, it can also be achieved by a non-specific alteration of the cell physiology or simply by the killing of this cell. Therefore, for a HTS application of the assay of the invention, it is critical to distinguish rapidly true inhibitors of a protein interaction from compounds that inhibit two-hybrid signals because of their toxicity.

The ability of assaying sequentially the activity of both luciferases in a same well and the extreme sensitivity of the above-described two-hybrid assay prompted the inventors to test the possibility of measuring in a single well *luc* and *ruc* two-hybrid signals produced by two yeast populations hosting to distinct protein interactions. As shown in figure 5, 10µL yeast suspensions still produced strong *luc* and *ruc* signals, readily detected in the same well. The addition of either FK506 or T3 produced the selective inhibition of the cognate protein interaction. The addition of kanamycin, a non-toxic yeast permeable molecule, affected neither *luc* nor *ruc* signals, whereas the addition of cycloheximide, a molecule known to be toxic for yeast, strongly inhibited both two-hybrid signals (Figure 5).

Yeast permeability is often considered as a major issue for drug screening. The *ERG*6 gene product participates in the biosynthesis of ergosterol, a major constituent of fungal membranes. Many studies have shown that *ERG*6 deleted yeast strains are more permeable to a wide diversity of small molecules. To increase the sensitivity of the screening assay of the invention, a targeted disruption of the *ERG*6 gene was performed.

As shown in Figure 5, the *ERG*6 deleted strain registered higher inhibitions of both protein interactions in response to FK506 or T3, as could be expected from an increased permeability to small molecules. It is worth noting that the two-hybrid signals produced in the *ERG*6 deleted strain were significantly lower than those produced in the parental strain (data not shown). Moreover, in this strain, FK506 induced an unexpected 40% inhibition of the *ruc* two-hybrid signal produced by the interaction between Trα and NCoR. This inhibition was probably caused by a non-specific toxic effect of a high concentration of FK506 on the *ERG*6 strain, which is generally less robust than its parental strain.

These experiments establish that two-hybrid assay above-described can be used in HTS for the discovery of small molecule inhibitors of protein interactions.

Discussion

In order to carry out the screening method of the invention, the inventors have designed a yeast two-hybrid assay showing an outstanding level of sensitivity and dynamic range in its ability to detect protein-protein interactions. For this, they made use of luciferase reporter genes, known to support highly sensitive assays for different reasons. These experiments showed that the described luminescent two-hybrid assay is highly quantitative and produces signals that correlated well to the binding affinities between the protein pairs that were analyzed. The issue of penetrance of two-hybrid interaction phenotypes has been already discussed at length and depends on many different variables pertaining on the one hand to the identity of the bait and the prey, and on the other hand to the reporter genes and the detection methods that are used [Serebriiskii, 2000]. Because of the profound differences existing between different baits and preys, two-hybrid assays cannot be used to get precise binding affinity values for protein interactions in general. However, the results showed that for a given bait and for a set of closely related proteins such as peptide aptamers, the use of quantitative two-hybrid assays can predict relative binding affinities with a high accuracy.

Such assays are extremely useful for QSAR (quantitative structure-activity relationship) analysis on peptide aptamer and protein mutants, aiming at defining models of complexes formed between peptide aptamers and their target proteins. The superior sensitivity and dynamic range conferred by the use of luciferases allows such assays to be performed easily and quickly with a plate reader (instead of a flow cytometer for GFP reporters) and permits to gain more information from aptamer and target protein variants displaying a wider range of binding affinities.

The dual-luminescent two-hybrid assay described above presents the advantageous simplicity of a forward two-hybrid assay while circumventing the requirement of a tedious parallel screening to eliminate toxic compounds. It is designed to be run from start to end in liquid and in microwell plates and can thus be fully automated, in contrast with the majority of two screening assays described so far, which were performed on agar plates apart for a recently published liquid two-hybrid assay [Nieuwenhuijsen, 2003].

The high sensitivity of our assay confers two determinant advantages. First, it will be possible to perform screening in the 384 well format, and possibly in the 1536 well format for HTS applications. Second, the duration of the screening assay can be limited to a few hours, as compared to 1 or 2 days for the assays described so far. Besides the positive impact on the throughput, the rapidity of the disclosed assay will limit the risks of missing interesting compounds that would end up being metabolized by yeast or that would show long-term toxicity. Another interesting feature is the possibility to perform screening against two protein interactions at once, with no worries of assay calibration.

The use of luciferases as two-hybrid reporter genes is also particularly well suited to detect inhibition of protein interactions. Although luciferase half-life has not be determined in yeast, it is reasonable to speculate that it should not be dramatically different from the half-life in mammalian cells, estimated at 3h [Thompson, 1991]. This contrasts with β-galactosidase which has an estimated half-life in yeast of about 20h [Serebriiskii, 2000]. It is also possible to destabilize the luciferase proteins, in order to reduce their half-life, thus obtaining an even more sensitive assay. Destabilization can be achieved with the incorporation of destabilizing sequences such as PEST [Rechsteiner, 1996].

Another significant advantage in using luciferase reporters in forward two-hybrid assays is the possibility of screening molecules that can induce protein interactions, or at least enhance weak interactions. Stabilizing protein interactions or restoring interactions that are abolished by loss of function mutations (such as those affecting tumor suppressor genes) would offer great therapeutic perspectives for a wide variety of pathologies.

Finally, the key feature of the assay developed by the inventors lies in the possibility of conferring an advantage to the screened compounds that can be pre-incubated with the yeast suspensions for the desired time, before inducing the expression of the prey. This may increase significantly the hit rates of the screening, for different reasons.

First, it is conceivably "easier" for a small molecule to prevent two proteins from interacting than to disrupt already formed complexes, especially when said complexes are stable. A small molecule targeting the bait will be further advantaged in that it can occupy its acceptor site before the prey is even expressed.

Second, pre-incubating yeast with small molecules will increase the intracellular concentration of those compounds that penetrate yeast moderately. This may have a great impact on hit rates as yeast permeability is often considered as an issue for screening applications.

In conclusion, the inventors have developed a yeast two-hybrid assay ideally suited for high-throughput screening of small molecule modulators of protein interactions. It will successfully identify hits against a wide range of intracellular protein interactions that represent a highly exciting, largely untapped, reservoir of therapeutic targets.

Bibliography:

**Bai ,** C. and S.J. Elledge, *Searching for interacting proteins with the two-hybrid system I,* in *The Yeast Two-Hybrid System,* B. Bartel and S. Fields, Editors. 1997, Oxford University Press: New York, Oxford. p. 11-28.
**Cayrol,** C, Cabrolier G., Ducommun B., *Use of the two-hybrid system to identify protein-protein interaction temperature-sensitive mutants: application* to *the CDK2/p21Cip1 interaction.* Nucleic Acids Res. 1997; **25**(18):p. 3743.
**Clark,** D.D. and B.R. Peterson, *Analysis of Protein Tyrosine Kinase Inhibitors in Recombinant Yeast Lacking the ERG6 Gene.* Chembiochem, 2003. **4**(1): p. 101-7.
**Colas,** P., et al., *Genetic selection of peptide aptamers that recognize and inhibit cyclin- dependent kinase* 2. Nature, 1996. **380**(6574): p. 548-50.
**Colas,** P., et al., *Targeted modification and transportation of cellular proteins.* Proc Natl Acad Sci U S A, 2000a. **97**(25): p. 13720-5.
**Colas,** P., *Combinatorial protein reagents to manipulate protein function.* Curr Opin Chem Biol, 2000b. **4**(1): p. 54-9.
**Crabtree,** G.R., and Schreiber, S.L., Trends Biochem Sci 1996, **21**:p. 418.
**Finley,** R.L. Jr and Brent, R., *Interaction mating reveals binary and ternary connections between Drosophila cell cycle regulators.* Proc Natl Acad Sci U S A. 1994;**91** (26):p. 12980.
**Gietz,** D., et al., *Improved method for high efficiency transformation of intact yeast cells.* Nucleic Acids Res, 1992. **20**(6): p. 1425.
**Golemis,** E. and R. Brent, *Searching for Interacting Proteins with the Two-Hybrid System III,* in *The Yeast Two-*

*Hybrid System,* P.L. Bartel and S. Fields, Editors. 1997, Oxford University Press: Oxford. p. 43-72.

**Greer,** L.F., 3rd, et al., *Imaging of light emission from the expression of luciferases in living cells and organisms: a review.* Luminescence, 2002. **17**(1). p. 43-74.

**Huang,** J. and S.L. Schreiber, *A yeast genetic system for selecting small molecule inhibitors of protein-protein interactions in nanodroplets.* Proc Natl Acad Sci U S A, 1997. **94**(25): p. 13396-401.

**Nieuwenhuijsen,** B.W., et al., *A dual luciferase multiplexed high-throughput screening platform for protein-protein interactions.* J. Biomol. Screening, 2003, **8**(6): p. 676.

**Rechsteiner M,** Rogers SW, *PEST sequences and regulation by proteolysis.* Trends Biochem Sci. 1996, **21**(7):p. 267-71.

**Seol,** W., et al., *Two receptor interacting domains in the nuclear hormone receptor corepressor RIP13/N-CoR.* Mol Endocrinol, 1996. **10**(12): p. 1646-55.

**Serebriiskii,** I.G. and E.A. Golemis, *Uses of lacZ to study gene function: evaluation of beta-galactosidase assays employed in the yeast two-hybrid system.* Anal Biochem, 2000. **285**(1): p. 1-15.

**Sikorski,** R.S. and P. Hieter, *A system of shuttle vectors and yeast host strains designed for efficient manipulation of DNA in Saccharomyces cerevisiae.* Genetics, 1989. **122**(1): p. 19-27.

**Kato-Stankiewicz,** J., et al., *Inhibitors of Ras/Raf-1 interaction identified by two-hybrid screening revert Ras-dependent transformation phenotypes in human cancer cells.* Proc Natl Acad Sci U S A, 2002. **99**(22): p. 14398-403.

**Tagami,** T., et al., *Nuclear receptor corepressors activate rather than suppress basal transcription of genes that are negatively regulated by thyroid hormone.* Mol Cell Biol, 1997. **17**(5): p. 2642-8.

**Thompson,** J.F., L.S. Hayes, and D.B. Lloyd, *Modulation of firefly luciferase stability and impact* on *studies ofgene regulation.* Gene, 1991. **103**(2): p. 171-7.

**Vidal, M.,** et al., *Reverse two-hybrid and one-hybrid systems to detect dissociation of protein-protein and DNA-protein interactions.* Proc Natl Acad Sci U S A, 1996. **93**(19): p. 10315-20.

**Vidal,** M. and H. Endoh, *Prospects for drug screening using the reverse two-hybrid system.* Trends Biotechnol, 1999a. **17**(9): p. 374-81.

**Vidal**, M. and P. Legrain, *Yeast forward and reverse 'n'-hybrid systems.* Nucleic Acids Res, 1999b. **27**(4): p. 919-29.

**Vojtek,** A.B., J.A. Cooper, and S.M. Hollenberg, *Searching for interacting proteins with the two-hybrid system II,* in *The yeast two-hybrid system,* B. Bartel and S. Fields, Editors. 1997, Oxford University Press: New York, Oxford. p. 29-42.

**Wang,** T., P.K. Donahoe, and A.S. Zervos, *Specific interaction of type I receptors of the TGF-beta family with the immunophilin FKBP-12.* Science, 1994. **265**(5172): p. 674-6.

**Wang,** Y., et al., *Regulator of G protein signaling Z1 (RGSZ1) interacts with Galpha i subunits and regulates Galpha i-mediated cell signaling.* J Biol Chem, 2002. **277**(50): p. 48325-32. Epub 2002 Oct 11.

**Young,** K., et al., *Identification of a calcium channel modulator using a high throughput yeast two-hybrid screen.* Nat Biotechnol, 1998. **16**(10): p. 946-50.

## Claims

1. Intracellular screening method for detecting exogenous candidate compounds C capable of modulating the intracellular interaction between two peptidic binding partners A and B, or of creating such an interaction, said intracellular interaction being responsible for a given specific phenotype, wherein

   - in a first step, the candidate compound C is allowed to contact the binding partner A, inside a cell, in the absence of the partner B,

   - in a second step, the partner B is then made available for binding to partner A, and

   - in a third step, the phenotype of said cell is detected.

2. Screening method according to claim 1, wherein any modification in the binding partner interaction is detectable by a modification in said specific phenotype.

3. Screening method according to claim 1 or 2, wherein the candidate compound modulates the interaction by preventing, disrupting, modifying or stabilizing the intracellular interaction.

4. Screening method according to daim 1 or 2, wherein the candidate compound creates the interaction by generating or restoring an intracellular interaction.

**5.** Screening method according to daim 1 wherein said specific phenotype is due to the expression of a reporter gene.

**6.** Screening method according to claim 1, wherein said given specific phenotype is the phenotype exhibited by the cell when no candidate compound C is allowed to contact said binding partner A.

**7.** Screening method according to any one of daims 1 to 6, wherein said binding partners are components of a two-hybrid system.

**8.** Screening method according to any one of daims 1 to 7, wherein partner B is made available for binding, subsequent to contacting of partner A and compound C, by spatial and/or temporal regulation of availability of B.

**9.** Screening method according to claim 8, wherein said second interacting partner B is made available by induction of its expression.

**10.** Screening method according to claim 8, wherein said second interacting partner B bears a thermo-sensitive mutation that prevents binding to partner A at restrictive temperature and is made available by switching the temperature from a restrictive to a permissive one.

**11.** Screening method according to daim 8, wherein said second interacting partner B is sequestered in an intracellular compartment where the other partner A is not present and is made available by being released from said compartment.

**12.** Screening method according to any one of daims 1 to 7, wherein said second interacting partner B is made available by direct introduction into the cell.

**13.** Screening method according to any one of daims 1 to 7, wherein said second interacting partner B is made available by cell fusion or mating.

**14.** Screening method according to daim 1, wherein said candidate compound C is non-peptidic.

**15.** Screening method according to claim 14, wherein said non peptidic compound C is a small organic molecule, an inorganic compound, a metabolic substance or a nudeotidic molecule (DNA or RNA).

**16.** Screening method according to daim 1, wherein said candidate compound C is a conformationally-constrained peptide.

**17.** Screening method according to anyone of claims 1 to 16, wherein said binding partners A and B are different.

**18.** Screening method according to anyone of claims 1 to 17, wherein at least one of said binding partners is a protein complex.

**19.** Screening method according to any one of claims 1 to 18, wherein said cell is a yeast cell.

**20.** Screening method according to claim 19, wherein said yeast is *Saccharomyces cerevisiae.*

**21.** Screening method according to any one of daims 1 to 18, wherein said cell is a mammalian cell.

**22.** Screening method according to any one of claim 5, wherein said reporter gene is a luciferase gene.

**23.** Screening method according to any one of claims 1 to 13, wherein at least one of said binding partners is conformationally-constrained.

**24.** Screening method according to any one of claims 1 to 23 further comprising an additional step of isolating the compound C capable of modifying said given specific phenotype.

**25.** Screening method according to claim 24, further comprising an additional step of producing the compound C on a large scale.

26. Intracellular screening method for detecting exogenous candidate compounds C capable of modulating the intracellular interaction between two peptidic binding partners A and B and being incapable of modulating the interaction between peptidic binding partners A' and B', said intracellular interactions being responsible for given specific cellular phenotypes, wherein

- in a first step, the candidate compound C is allowed to contact the binding partner A, inside a cell, in the absence of the partner B, and is also allowed to contact the binding partner A' inside a cell, in the absence of partner B'

- in a second step, the partners B and B' are then made available for binding with their respective partners A and A', and

- in a third step, the cellular phenotypes are detected.

27. Screening method according to claim 26, wherein the partners A and A' are identical.

28. Screening method according to claim 26 or 27, wherein the partners B and B' are identical.

29. Screening method according to claim 26, wherein the partners A and B' are identical and partners B and A' are identical.

30. Screening method according to claim 26, wherein the interactions between peptidic binding partners A and B, and binding partners A' and B' occur in the same cell and wherein said given specific phenotypes are two different phenotypes.

31. Screening method according to claim 26, wherein the interactions between peptidic binding partners A and B, and binding partners A' and B' occur in two different cells.

32. Screening method according to claim 26, wherein said given specific phenotypes are two different phenotypes.

33. Screening method according to claim 31, wherein the two different cells are mixed together in the same sample.

34. Screening method according to any one of claims 26 to 33, wherein said given specific phenotype(s) is (are) due to expression of one or more reporter genes.

35. Screening method according to daim 34, wherein said reporter genes are two different luciferase genes, for example *luc* from *Photinus pyralis* and *ruc* from *Renilla reniformis.*

36. Screening method according to claim 34, wherein said reporter genes are two different variants of the Green fluorescent protein gene.

37. Screening method according to claim 26, wherein said exogenous candidate compound is also assayed for its capacity to modulate at least one further intracellular interaction between two peptidic binding partners A" and B", said interaction being responsible for a further given specific phenotype, wherein

- in the first step, candidate compound C is also allowed to contact partner A", inside a cell, in the absence of the partner B";

- in the second step, partner B" is made available for binding with partner A",

- in the third step, the further phenotype is also detected.

38. Screening method according to any one of daims 1 to 37, wherein the binding partners A and B, or A' and B', or A" and B", are chosen among the group of binding partners consisting of: TGFβ-R and FKBP12; TGFβ-R and FK506; FKBP12 and FK506; TR-α and NCoR.

39. Screening method according to any one of claims 1 to 38 which is carried out in liquid medium.

# Figure 1

# Figure 2

EP 1 582 590 A1

# Figure 3A

# Figure 3B

EP 1 582 590 A1

# Figure 4A

# Figure 4B

# Figure 5

**PARTIAL EUROPEAN SEARCH REPORT**     Application Number

which under Rule 45 of the European Patent Convention EP 04 29 0877
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 2003/211523 A1 (ZHANG SHUANG ET AL) 13 November 2003 (2003-11-13) * the whole document * ----- | 1-39 | C12N15/10 G01N33/50 C12Q1/68 |
| X | WO 02/50259 A (NATIONAL RESEARCH COUNCIL OF CANADA; ZHAO, HUI-FEN; SHEN, SHI-HSIANG) 27 June 2002 (2002-06-27) * the whole document * ----- | 1-39 | |
| X | US 5 885 779 A (SADOWSKI ET AL) 23 March 1999 (1999-03-23) * the whole document * ----- | 1-39 | |
| D,X | COLAS P ET AL: "TARGETED MODIFICATION AND TRANSPORTATION OF CELLULAR PROTEINS" PROCEEDINGS OF THE NATIONAL ACADEMY OF SCIENCES OF USA, NATIONAL ACADEMY OF SCIENCE. WASHINGTON, US, vol. 97, no. 25, 5 December 2000 (2000-12-05), pages 13720-13725, XP001032669 ISSN: 0027-8424 * the whole document * ----- -/-- | 1-39 | TECHNICAL FIELDS SEARCHED (Int.Cl.7) C12N G01N C12Q |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 13 April 2005 | Pinheiro Vieira, E |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
................................................................
& : member of the same patent family, corresponding
document

EPO FORM 1503 03.82 (P04C07)

**EP 1 582 590 A1**

**European Patent Office**

**PARTIAL EUROPEAN SEARCH REPORT**

Application Number

EP 04 29 0877

## DOCUMENTS CONSIDERED TO BE RELEVANT

| Category | Citation of document with indication, where appropriate, of relevant passages | Relevant to claim |
|---|---|---|
| X | COLAS P ET AL: "The impact of two-hybrid and related methods on biotechnology" TRENDS IN BIOTECHNOLOGY, ELSEVIER PUBLICATIONS, CAMBRIDGE, GB, vol. 16, no. 8, 1 December 1998 (1998-12-01), pages 355-363, XP004131483 ISSN: 0167-7799 * the whole document * | 1-39 |

-----

**CLASSIFICATION OF THE APPLICATION (Int.Cl.7)**

**TECHNICAL FIELDS SEARCHED (Int.Cl.7)**

EPO FORM 1503 03.82 (P04C10)

30

**European Patent Office**

**INCOMPLETE SEARCH
SHEET C**

Application Number

EP 04 29 0877

Although claims 1-18, 21 and  26-39 are directed to a diagnostic method practised on the human/animal body (Article 52(4) EPC), the search has been carried out and based on the alleged effects of the compound/composition.

-----

**ANNEX TO THE EUROPEAN SEARCH REPORT
ON EUROPEAN PATENT APPLICATION NO.**

EP 04 29 0877

This annex lists the patent family members relating to the patent documents cited in the above-mentioned European search report.
The members are as contained in the European Patent Office EDP file on
The European Patent Office is in no way liable for these particulars which are merely given for the purpose of information.

13-04-2005

| Patent document cited in search report | Publication date | Patent family member(s) | Publication date |
|---|---|---|---|
| US 2003211523 A1 | 13-11-2003 | NONE | |
| WO 0250259 A | 27-06-2002 | AT 269899 T | 15-07-2004 |
| | | AU 1575402 A | 01-07-2002 |
| | | WO 0250259 A2 | 27-06-2002 |
| | | CA 2431898 A1 | 27-06-2002 |
| | | DE 60104019 D1 | 29-07-2004 |
| | | EP 1343882 A2 | 17-09-2003 |
| | | ES 2223736 T3 | 01-03-2005 |
| | | US 2004067483 A1 | 08-04-2004 |
| US 5885779 A | 23-03-1999 | AU 748706 B2 | 13-06-2002 |
| | | AU 9057398 A | 29-03-1999 |
| | | CA 2303416 A1 | 18-03-1999 |
| | | WO 9913069 A1 | 18-03-1999 |
| | | EP 1012265 A1 | 28-06-2000 |
| | | JP 2001515718 T | 25-09-2001 |

EPO FORM P0459

For more details about this annex : see Official Journal of the European Patent Office, No. 12/82

SEQUENCE LISTING

<110>  APTANOMICS

<120>  SEQUENTIAL INTRACELLULAR SCREENING METHOD

<130>  B5733 - CA/CS/PAM

<140>  EP 04290877.2
<141>  2004-04-02

<160>  12

<170>  PatentIn version 3.1

<210>  1
<211>  34
<212>  DNA
<213>  Artificial

<220>
<223>  Primer

<400>  1
tatataccgc ggcatatcca tatctaatct tacc                                    34


<210>  2
<211>  34
<212>  DNA
<213>  Artificial

<220>
<223>  Primer

<400>  2
taagacgccg gcgatatcaa aaaagaggaa ctgc                                    34


<210>  3
<211>  30
<212>  DNA
<213>  Artificial

<220>
<223>  Primer

<400>  3
ccgactcgag aagctttgga cttcttcgcc                                          30


<210>  4
<211>  32
<212>  DNA
<213>  Artificial

<220>
<223>  Primer

<400>  4
ggggtaccgt gtggaagaac gattacaaca gg                                       32

```
<210>  5
<211>  45
<212>  DNA
<213>  Artificial

<220>
<223>  Primer

<400>  5
ataagaatgc ggccgcatgg aagacgccaa aaacataaag aaagg          45


<210>  6
<211>  50
<212>  DNA
<213>  Artificial

<220>
<223>  Primer

<400>  6
aagaagtcca aagcttctcg agttacaatt tggactttcc gcccttcttg      50


<210>  7
<211>  39
<212>  DNA
<213>  Artificial

<220>
<223>  Primer

<400>  7
ataagaatgc ggccgcatga cttcgaaagt ttatgatcc                 39


<210>  8
<211>  53
<212>  DNA
<213>  Artificial

<220>
<223>  Primer

<400>  8
aagaagtcca aagcttctcg agttattgtt catttttgag aactcgctca acg  53


<210>  9
<211>  31
<212>  DNA
<213>  Artificial

<220>
<223>  Primer

<400>  9
ccactttaac taatactttc aacattttcg g                          31
```

```
<210>  10
<211>  21
<212>  DNA
<213>  Artificial

<220>
<223>  Primer

<400>  10
caagccgaca accttgattg g                                          21


<210>  11
<211>  19
<212>  DNA
<213>  Artificial

<220>
<223>  Primer

<400>  11
cgggtgtttt ctcctatcc                                             19


<210>  12
<211>  20
<212>  DNA
<213>  Artificial

<220>
<223>  Primer

<400>  12
tgtttgtaag gcctgctagc                                            20
```